# EUROPÄISCHE PATENTSCHRIFT

(11) **EP 1 515 936 B1**
(45) Veröffentlichungstag und Bekanntmachung des Hinweises auf die Patenterteilung: **18.03.2009**
(21) Anmeldenummer: 03738044.1
(22) Anmeldetag: 17.06.2003
(51) Int. Cl.: C07C 41/06, C07B 37/02, C07C 67/04, C07C 29/46

(54) **VERFAHREN ZUR TELOMERISATION VON 1,3-BUTADIEN**
METHOD FOR THE TELOMERISATION OF 1,3-BUTADIENE
PROCEDE POUR LA TELOMERISATION DE 1,3-BUTADIENE

(30) Priorität: 29.06.2002 DE 10229290; 22.03.2003 DE 10312829
(43) Veröffentlichungstag der Anmeldung: 23.03.2005
(73) Patentinhaber: Evonik Oxeno GmbH, 45772 Marl (DE)
(72) Erfinder: RÖTTGER, Dirk, 45657 Recklinghausen (DE); JACKSTELL, Ralf, 27472 Cuxhaven (DE); KLEIN, Holger, 18069 Rostock (DE); BELLER, Matthias, 18211 Nienhagen (DE)
(74) Vertreter: Hirsch, Hans-Ludwig
(86) Internationale Anmeldenummer: PCT/EP2003/006356
(87) Internationale Veröffentlichungsnummer: WO 2004/002931

(56) Entgegenhaltungen:
- WO-A-02/100803
- JACKSTELL R ET AL: "A HIGHLY EFFICIENT CATALYST FOR THE TELOMERIZATION OF 1,3-DIENES WITH ALCOHOLS: FIRST SYNTHESIS OF A MONOCARBENEPALLADIUM(0)-OLEFIN COMPLEX" ANGEWANDTE CHEMIE. INTERNATIONAL EDITION, VERLAG CHEMIE. WEINHEIM, DE, Bd. 41, Nr. 6, 15. März 2002 (2002-03-15), Seiten 986-989, XP001111654 ISSN: 0570-0833

## Beschreibung

Die vorliegende Erfindung betrifft ein Verfahren zur Telomerisation von 1,3-Butadien mit Nucleophilen (II) wobei als Katalysator ein Metall-Carben-Komplex eingesetzt wird.

Unter Telomerisation wird im Rahmen dieser Erfindung die Umsetzung von Olefinen mit konjugierten Doppelbindungen (konjugierte Diene) in Gegenwart eines Nucleophils (Telogens) verstanden. Als Hauptprodukte werden dabei Verbindungen erhalten, die sich aus zwei Äquivalenten des Diens und einem Äquivalent des Nucleophils aufbauen.

Die Produkte der Telomerisationsreaktion haben als vielseitig einsetzbare Vorstufen für Lösemittel, Weichmacher, Feinchemikalien und Wirkstoffvorprodukte technische Bedeutung. Die aus Butadien erhältlichen Verbindungen Octadienol, Octadienylether oder Octadienylester sind potentielle Zwischenprodukte in Verfahren zur Darstellung von entsprechenden Alkenen.

Die Telomerisation von Dienen mit Nucleophilen ist eine technisch interessante Methode zur Veredelung von kostengünstigen, industriell verfügbaren Dienen. Von besonderem Interesse ist aufgrund der guten Verfügbarkeit die Verwendung von Butadien, Isopren oder von diese Diene enthaltenden Cracker-Schnitten. Bis dato wird die Telomerisation von Butadien jedoch lediglich von der Firma Kuraray im Feinchemikalienbereich zur Synthese von 1-Octanol praktisch angewendet. Gründe, die den breiteren Einsatz von Telomerisationsprozessen verhindern, sind unter anderem mangelnde Katalysatoraktivitäten, Katalysatorproduktivitäten und Selektivitätsprobleme von Telomerisationskatalysatoren. Somit führen die bekannten Telomerisationsprozesse zu hohen Katalysatorkosten und/oder Nebenprodukten, die eine großtechnische Realisierung verhindern.

Als wirksame Katalysatoren für die Telomerisation haben sich unter anderem halogenfreie Palladium(0)- sowie Palladium(II)-Verbindungen erwiesen (A. Behr, in "Aspects of Homogeneous Catalysis"; Herausgeber R. Ugo, D. Reidel Publishing Company, Doordrecht/Boston/Lancaster, 1984, Vol. 5, 3). Daneben wurden auch Verbindungen anderer Übergangsmetalle, wie z. B. Cobalt (R. Baker, A. Onions, R. J. Popplestone, T.N. Smith, J. Chem. Soc., Perkin Trans. II 1975, 1133-1138), Rhodium, Nickel (R. Baker, D.E. Halliday, T.N. Smith, J. Organomet. Chem. 1972, 35, C61-C63; R. Baker, Chem. Rev. 1973, 73, 487-530; R. Baker, A.H. Cook, T.N Smith, J. Chem. Soc., Perkin Trans. II 1974, 1517-1524.) und Platin, als Katalysatoren eingesetzt.

Die Telomerisation von Dienen ist in der Fachliteratur ausführlich beschrieben. Die oben genannten Katalysatoren liefern beispielsweise bei der Telomerisation von Butadien mit Methanol generell Gemische der aufgeführten Produkte **1a, 1b, 2, 3** mit X = O, R^{a} = Me. Hauptprodukte sind dabei die gewünschten technisch wichtigen linearen Telomere **1a** und **1b.** Jedoch entstehen signifikante Anteile des verzweigten Telomers **2** und von 1,3,7-Octatrien 3**.**

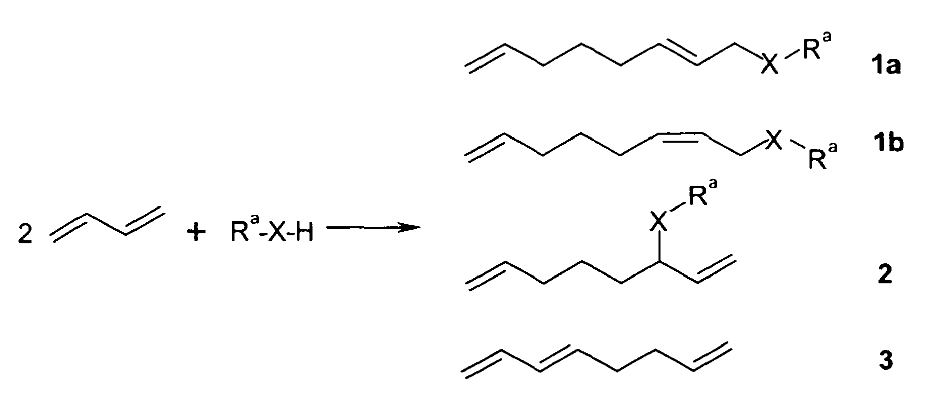

Weiterhin entstehen 4-Vinyl-1-cyclohexen (Diels-Alder-Produkt des Butadiens) in variablen Ausbeuten sowie - in der Regel in nur geringen Mengen - weitere Nebenprodukte. Dieses Spektrum von Produkten findet man generell auch bei Einsatz anderer Nucleophile mit aktiven H-Atomen, wobei an Stelle der Methoxygruppe die entsprechenden Reste des jeweiligen Nucleophils treten.

Die signifikante Bildung der genannten Nebenprodukte ist ein weiterer Grund, der eine Umsetzung eines wirtschaftlichen und umweltfreundlichen Verfahrens außerordentlich schwierig macht. So konnten, obwohl die Telomerisation von Butadien mit Methanol bereits von mehreren Firmen intensiv bearbeitet und patentiert wurde, die oben genannten Probleme nicht befriedigend gelöst werden.

In einem von Dow Chemical in WO 91/09822 im Jahr 1989 beschriebenen kontinuierlichen Verfahren mit Palladiumacetylacetonat/Triphenylphosphan als Katalysator wurden Katalysatorproduktivitäten (tumover numbers) bis zu 44000 erzielt. Allerdings sind die Chemoselektivitäten bei derartigen Katalysatorumsatzzahlen für das Zielprodukt 1 < 85 %.

National Distillers and Chem. Corp. (US 4,642,392, US 4,831,183) beschrieben 1987 ein diskontinuierliches Verfahren zur Herstellung von Octadienylethern. Dabei wurde das Produktgemisch destillativ vom Katalysator (Palladiumacetat / 5 Äq. Triphenylphosphan) abgetrennt, der in Tetraglyme gelöst zurückbleibt. Der Katalysator kann bis zu zwölfmal wiederverwendet werden, wobei jeweils Phosphan ergänzt wird. Der Startansatz lieferte den linearen Ether allerdings in nur 57 % Ausbeute (entspricht TON 2000). Das n/iso-Verhältnis von Produkt 1 zu Produkt 2 beträgt in diesem Fall nur 3.75 : 1. In einem weiteren Patent von National Distillers wurde das Produktgemisch durch Extraktion mit Hexan von der Reaktionslösung abgetrennt. Die Telomerisation wurde dabei in Dimethylformamid oder Sulfolan mit dem Katalysatorgemisch Palladim(II)acetat / 3 Äq. Triphenylphosphinmonosulfonat durchgeführt. Der erste Ansatz lieferte das lineare Telomer mit einer TON von 900. Die Selektivität bezüglich des linearen Alkohols betrug geringe 40 %.

Auch längerkettige primäre Alkohole wie Ethanol, Propanol und Butanol (J. Beger, H. Reichel, J. Prakt. Chem. 1973, 315, 1067) bilden mit Butadien die entsprechenden Telomere. Allerdings ist die Katalysatoraktivität der bekannten Katalysatoren hier noch geringer als in den oben genannten Fällen. So wurden unter identischen Reaktionsbedingungen [Pd(acetylacetonat)₂ / PPh₃ / Butadien / Alkohol = 1 : 2 : 2000 : 5000; 60 °C / 10 h] die Telomere von Methanol mit 88 % Ausbeute, diejenigen von Propanol mit 65 % Ausbeute und von Nonanol nur noch mit 21 % Ausbeute gebildet.

Zusammenfassend kann man sagen, dass die bekannten Palladiumphosphankatalysatoren für Telomerisationsreaktionen von Butadien mit Alkoholen keine befriedigenden Selektivitäten von > 95 % Chemo- und Regioselektivität erreicht werden, um ein ökologisch vorteilhaftes Verfahren zu erzielen.

Carbonsäuren sind wie Alkohole geeignete Nucleophile in Telomerisationsreaktionen. Aus Essigsäure und Butadien erhält man in guten Ausbeueten die entsprechenden Octadienylderivate **1a, 1b** und **2** mit R^{a} = Me-CO, X = O (DE 2 137 291). Das Verhältnis der Produkte 1/2 kann über die Liganden am Palladium beeinflusst werden (D. Rose, H. Lepper, J. Organomet. Chem. 1973, 49, 473). Mit Triphenylphosphin als Ligand wurde ein Verhältnis 4/1 erreicht, bei Einsatz von Tris(o-methylphenyl)phosphit konnte das Verhältnis auf 17/1 gesteigert werden. Andere Carbonsäuren wie Pivalinsäure, Benzoesäure oder Methacrylsäure, aber auch Dicarbonsäuren lassen sich ebenfalls mit Butadien umsetzen.

Shell Oil hat aufbauend auf die Telomerisation von konjugierten Dienen mit Carbonsäuren ein Verfahren zur Herstellung von α-Olefinen in der US 5 030 792 beschrieben.

Telomerisationsreaktionen, bei denen Wasser als Nucleophil eingesetzt wird, sind unter anderem von der Firma Kuraray intensiv untersucht worden (US 4 334 117, US 4 356 333, US 5 057 631). Dabei werden Phosphine, meistens wasserlösliche Phosphine, oder Phosphoniumsalze (EP 0 296 550) als Liganden eingesetzt. Der Einsatz von wasserlöslichen Diphosphinen als Ligand wird in WO 98/08 794 beschrieben, DE 195 23 335 offenbart die Umsetzung von Alkadienen mit Wasser in Gegenwart von Phosphonit oder Phosphinitliganden.

Die Telomerisation von Butadien mit Nucleophilen, wie Formaldehyd, Aldehyden, Ketonen, Kohlendioxid, Schwefeldioxid, Sulfinsäuren, β-Ketoestern, β-Diketonen, Malonsäureestern, α-Formylketonen und Silanen ist ebenfalls beschrieben.

Der größere Teil der Arbeiten zur Telomerisation wurde mit Butadien durchgerührt. Die Reaktion ist aber auch auf andere Diene mit konjugierten Doppelbindungen anwendbar. Diese kann man formal als Derivate des Butadiens betrachten, in dem Wasserstoffatome durch andere Gruppen ersetzt sind. Technisch bedeutsam ist vor allem Isopren. Da Isopren im Gegensatz zum Butadien ein unsymmetrisches Molekül ist, kommt es bei der Telomerisation zur Bildung von weiteren Isomeren (J. Beger, Ch. Duschek, H. Reichel, J. Prakt. Chem. 1973, 315, 1077 - 89). Das Verhältnis dieser Isomeren wird dabei erheblich durch die Art des Nucleophils und auch die Wahl der Liganden beeinflusst.

In dem Artikel von R. Jackstell et al. in Angew. Chemie Int. Ed. 41, 6, Seiten 986 - 989 (2002) wird eie Telomerisation von 1,3-Butadien mit Methanol als Nucleophil in Gegenwart eines Monocarben-Pd(O)-Komplexes beschrieben, deren Struktur dort näher spezifiziert ist (Figur 1).

Aufgrund der genannten Bedeutung der Telomerisationsprodukte und den Problemen des derzeitigen Stands der Technik, besteht ein großer Bedarf nach neuen Katalysatorsystemen für Telomerisationsreaktionen, die mit einer hohen Katalysatorproduktivität für die großtechnische Durchführung geeignet sind und die Telomerisationsprodukte in hoher Ausbeute und Reinheit liefern.

Überraschender Weise wurde gefunden, das Telomerisationsreaktionen eines nicht cyclischen Olefins mit einem Nucleophil durch Metalle der 8. bis 10. Gruppe des Periodensystems und bestimmten Carbenliganden mit hohen Umsätzen und Selektivitäten katalysiert werden.

Gegenstand der Erfindung ist daher ein Verfahren zur katalytischen Telomerisation 1,3-Butadien mit mindestens einem Nucleophil,
wobei als Katalysator Komplexe eingesetzt werden, die Metalle der 8. bis 10. Gruppe des Periodensystems der Elemente und mindestens einen Carbenligand nach einer der allgemeinen Formeln 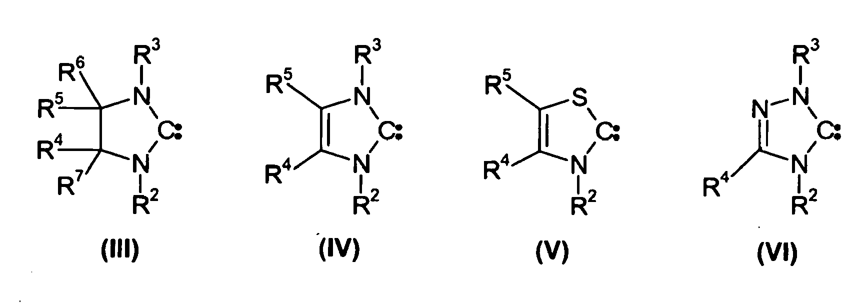 enthalten, mit
- R²; R³:: gleich oder verschieden a) lineare, verzweigte, substituierte oder unsubstituierte cyclische oder alicyclische Alkylgruppen mit 1 bis 24 Kohlenstoffatomen,
oder b) substituierte oder unsubstituierte, mono- oder polycyclische Arylgruppen mit 6 bis 24 Kohlenstoffatomen
oder c) mono- oder polycyclischer, substituierter oder unsubstituierter Heterocyclus mit 4 bis 24 Kohlenstoffatomen und mindestens einem Heteroatom aus der Gruppe N, O, S
- R⁴, R⁵, R⁶ R⁷:: gleich oder verschiedenen
Wasserstoff, Alkyl, Aryl, Heteroaryl, -CN, -COOH, -COO-Alkyl-, -COO-Aryl-, -OCO-Alkyl-, -OCO-Aryl-, -OCOO-Alkyl-, -OCOO-Aryl-, -CHO, -CO-Alkyl-, -CO-Aryl-, -O-Alkyl-, -O-Aryl-, -NH₂, -NH(Alkyl)-, -N(Alkyl)₂-, -NH(Aryl)-, -N(Alkyl)₂-, -F, -Cl, -Br, -I, -OH, -CF₃, -NO₂, -Ferrocenyl, -SO₃H, -PO₃H₂, wobei die Alkylgruppen 1 - 24 und die Arylgruppen 5 bis 24 Kohlenstoffatome beinhalten und die Reste R⁴ und R⁵ auch Teil eines verbrückenden aliphatischen oder aromatischen Ringes sein können,
mit der Maßgabe, das in Kombination mit Pd als Metall der 8. bis 10. Gruppe des Periodensystems R² und/oder R³ die Bedeutung c) hat.

R² und R³ stehen insbesondere für einen mono- oder polycyclischen Ring, der mindestes ein Heteroatom ausgewählt aus den Elementen Stickstoff, Sauerstoff und Schwefel enthält und gegebenenfalls weitere Substituenten ausgewählt aus den Gruppen -CN, -COOH, -COO-Alkyl-, -COO-Aryl-, -OCO-Alkyl-, -OCO-Aryl-, -OCOO-Alkyl-, -OCOO-Aryl-, -CHO; -CO-Alkyl-, -CO-Aryl-, -Aryl-, -Alkyl-, -O-Alkyl-, -O-Aryl-, -NH₂, -NH(Alkyl)-, -N(Alkyl)₂-, -NH(Aryl)-, -N(Alkyl)₂-, -F, -Cl, -Br, -I, -OH, -CF₃, -NO₂ -Ferrocenyl, -SO₃H, -PO₃H₂ aufweist. Die Alkylgruppe weisen 1 bis 24 und die Arylgruppen 5 bis 24 Kohlenstoffatome auf. In dem Fall, das Pd als Metall der 8. bis 10. Gruppe des Periodensystems verwendet wird, weist einer oder beide Liganden R² und R³, diese Bedeutungen auf.

Die Reste R², R³, R⁴, R⁵, R⁶ und/oder R⁷ können jeweils gleich oder verschieden sein und mindestens einen Substituenten aus der Gruppe -H, -CN, -COOH, -COO-Alkyl, -COO-Aryl, -OCO-Alkyl, -OCO-Aryl, -OCOO-Alkyl, -OCOO-Aryl, -CHO, -CO-Alkyl, -CO-Aryl, -Aryl, -Alkyl, -Alkenyl, -Allyl, -O-Alkyl, -O-Aryl, -NH₂, -NH(Alkyl), -N(Alkyl)₂, -NH(Aryl), -N(Alkyl)₂, -F, -Cl, -Br, -I, -OH, -CF₃, -NO₂, -Ferrocenyl, -SO₃H, -PO₃H₂ aufweisen, wobei die Alkylgruppen 1 bis 24, bevorzugt 1 bis 20, die Alkenylgruppen 2 bis 24, die Alylgruppen 3 bis 24 und die mono- oder polycyclischen Arylgruppen 5 bis 24 Kohlenstoffatome beinhalten.

Die Reste R⁴ bis R⁶ können z. B. über (CH₂)- oder (CH)-Gruppen miteinander kovalent verknüpft sein.

Substituenten mit aciden Wasserstoffatomen können an Stelle der Protonen auch Metall- oder Ammoniumionen aufweisen.

Die Reste R² und R³ stehen unter anderem für mono- oder polycyclische Ringe, die mindestens ein Heteroatom enthalten. Dies sind beispielsweise Reste, die sich von fünf und sechsgliedrigen Heteroalkanen, Heteroalkenen und Heteroaromaten wie 1,4-Dioxan, Morpholin, γ-Pyran, Pyridin, Pyrimidin, Pyrazin, Pyrrol, Furan, Thiophen, Pyrazol, Imidazol, Thiazol und Oxazol ableiten. In der nachfolgenden Tabelle sind konkrete Beispiele für derartige Reste R² und R³ widergegeben. Darin bezeichnet - jeweils den Anknüpfungspunkt zum Fünfring-Heterozyklus.

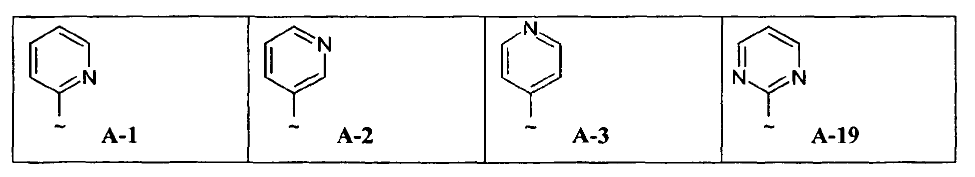

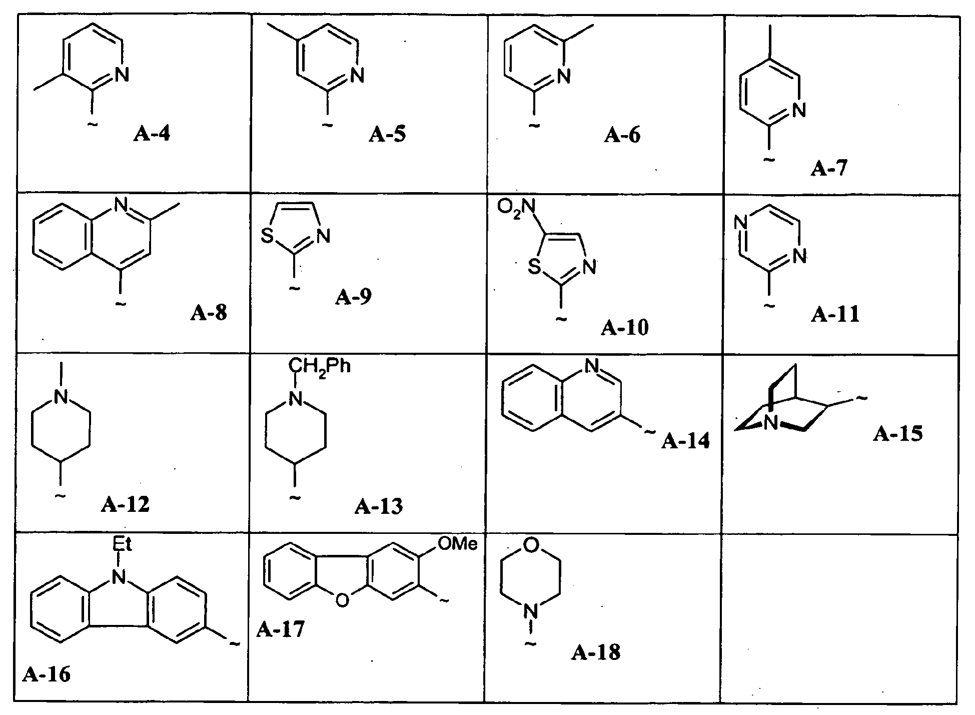

Im Rahmen dieser Erfindung werden unter Carbenliganden sowohl freie Carbene, die als Ligand fungieren können, als auch an Metall koordinierte Carbene verstanden.

Geeignete Metalle können z. B. Pd, Fe, Ru, Os, Co, Rh, Ir, Ni, oder Pt sein.

In der Telomerisation gemäß dem erfindungsgemäßen Verfahren wird 1,3-Butadien eingesetzt. Dabei können sowohl das reine Dien als auch Mischungen, die dieses Dien enthalten, eingesetzt werden.

Als 1,3-Butadien enthaltende Mischungen kommen vorzugsweise Mischungen von 1,3-Butadien mit anderen C3-, C4-Kohlenwasserstoffen und/oder C5-Kohlenwasserstoffen zum Einsatz. Solche Mischungen fallen beispielsweise bei Spalt(Crack)-Prozessen zur Produktion von Ethen an, in denen Raffineriegase, Naphtha, Gasöl, LPG (liquified petroleum gas), NGL (natural gas liquid) usw. umgesetzt werden. Die bei diesen Prozessen als Nebenprodukt anfallenden C₄-Schnitte enthalten je nach Crack-Verfahren unterschiedliche Mengen an 1,3-Butadien. Typische 1,3-Butadienkonzentrationen im C₄-Schnitt, wie sie aus einem Naphtha-Steamcracker erhalten werden, liegen bei 20 bis 70 % 1,3-Butadien.

Die C₄-Komponenten n-Butan, i-Butan, 1-Buten, cis-2-Buten, trans-2-Buten und i-Buten, die ebenfalls in diesen Schnitten enthalten sind, stören die Umsetzung im Telomerisationsschritt nicht oder nur unwesentlich.

Diene mit kumulierten Doppelbindungen (1,2-Butadien, Allen usw.) und Alkine, insbesondere Vinylacetylen, können hingegen als Moderatoren in der Telomerisationsreaktion wirken. Es ist daher vorteilhaft, die Alkine und ggf. das 1,2-Butadien vorher zu entfernen (z. B. gemäß DE 195 23 335). Dies kann, falls möglich, über physikalische Verfahren wie Destillation oder Extraktion erfolgen. Auf chemischem Weg können die Alkine über Selektivhydrierungen zu Alkenen oder Alkanen und die kumulierten Diene zu Monoenen reduziert werden. Verfahren für derartige Hydrierungen sind Stand der Technik und zum Beispiel in WO 98/12160, EP-A-0 273 900, DE-A-37 44 086 oder US 4 704 492 beschrieben.

Als Nukleophil werden im erfindungsgemäßen Verfahren bevorzugt Verbindungen der Formel **(II)**

R¹-Z-R¹ (II)

eingesetzt wird, mit
Z gleich O, N(R^{1"}),N(CH₂CH=CH₂), C(H₂), Si(R^{1"})(OH), C=O, C(H)(NO₂) oder S(O₂), also
-O- 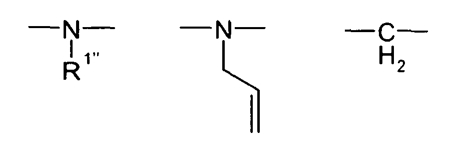 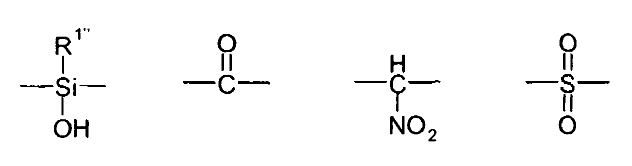 und R¹, R^{1'} oder R^{1"} gleich oder verschieden, H, substituierte oder unsubstituierte, lineare, verzweigte oder cyclische Alkylgruppen, Alkenylgruppen mit 1 bis 22 Kohlenstoffatomen, Carboxylgruppen oder Arylgruppen bedeuten und die Reste R¹, R^{1'} über kovalente Bindungen miteinander verknüpft sein können, wobei R¹ und R^{1'} gleich oder unterschiedlich substituiert sein können, z. B. mit einem oder mehreren Substituenten, ausgewählt aus der Gruppe -CN, -COOH, -COO-Alkyl, -CO-Alkyl, -Aryl, -Alkyl, -COO-Aryl, -CO-Aryl, -O-Alkyl, -O-CO-Alkyl, -N-Alkyl₂, -CHO, -SO₃H -NH₂, -F, -Cl, -OH, -CF₃, -NO₂. Die Alkylgruppen an den Substituenten weisen vorzugsweise 1 bis 24 und die Arylgruppen an den Substituenten weisen vorzugsweise 5 bis 24 Kohlenstoffatome auf.

In einer bevorzugten Ausführungsform werden als Nucleophil (II) Verbindungen der allgemeinen Formel (IIa) oder (IIb)

R¹-O-H (IIa),

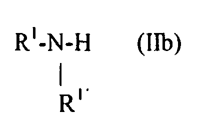

eingesetzt,
wobei R¹, R^{1'} jeweils gleich oder verschieden, H, substituierte oder unsubstituierte, lineare, verzweigte oder cyclische Alkylgruppen, Alkenylgruppen mit 1 bis 22 Kohlenstoffatomen, Carboxylgruppen oder Arylgruppen bedeuten und die Reste R¹, R^{1'} über kovalente Bindungen miteinander verknüpft sein können.

R¹ und R^{1'} können gleich oder unterschiedlich substituiert sein, z. B. mit einem oder mehreren Substituenten, ausgewählt aus der Gruppe -CN, -COOH, -COO-Alkyl, -CO-Alkyl, -Aryl, -Alkyl, -COO-Aryl, -CO-Aryl, -O-Alkyl, -O-CO-Alkyl, -N-Alkyl₂, -CHO, -SO₃H, -NH₂, -F, -Cl, -OH, -CF₃, -NO₂. Die Alkylgruppen weisen 1 bis 24 und die Arylgruppen 5 bis 24 Kohlenstoffatome auf.

Als Nucleophile werden bevorzugt alle Verbindungen eingesetzt, die der allgemeinen Formel (II) gerügen. Beispiele für Telogene nach der allgemeinen Formel (II) sind
- Wasser,
- Alkohole und Phenole wie zum Beispiel Methanol, Ethanol, n-Propanol, Isopropanol, Allylalkohol, Butanol, Octanol, 2-Ethylhexanol, Isononanol, Benzylalkohol, Cyclohexanol, Cyclopentanol, 2-Methoxyethanol, Phenol oder 2,7-Octadien-1-ol
- Dialkohole wie zum Beispiel Ethylenglycol, 1,2-propandiol, 1,3-Propandiol, 1,4-Butandiol, 1,2-Butandiol, 2,3-Butandiol und 1,3-Butandiol
- Polyole wie zum Beispiel Glycerin, Glucose, Saccharose,
- Hydroxyverbindungen wie zum Beispiel α-Hydroxyessigsäureester
- Carbonsäuren wie zum Beispiel Essigsäure, Propansäure, Butansäure, Isobutansäure, Benzoesäure, 1,2-Benzoldicarbonsäure, 1,3-Benzoldicarbonsäure, 1,4-Benzoldicarbonsäure, 1,2,4-Benzoltricarbonsäure,
- Ammoniak,
- primäre Amine wie zum Beispiel Methylamin, Ethylamin, Propylamin, Butylamin, Octylamin, 2,7-Octadienylamin, Dodecyclamin, Anilin, Ethylendiamin oder Hexamethylendiamin
- sekundäre Amine wie Dimethylamin, Diethylamin, N-Methylanilin, Bis(2,7-Octadienyl)amin, Dicyclohexylamin, Methylcyclohexylamin, Pyrrolidin, Piperidin, Morpholin, Piperazin oder Hexamethylenimin

Telogene, die selbst über eine Telomerisationsreaktion erhalten werden können, können direkt eingesetzt oder aber in situ gebildet werden. So kann beispielsweise 2,7-Octadien-1-ol aus Wasser und Butadien in Anwesenheit des Telomerisationskatalysators in situ gebildet werden, 2,7-Octadienylamin aus Ammoniak und 1,3-Butadien usw.

Besonders bevorzugt eingesetzte Telogene sind Wasser, Methanol, Ethanol, n-Butanol, Allylalkohol, 2-Methoxyethanol, Phenol, Ethylenglycol, 1,3-Propandiol, Glycerin, Glucose, Saccharose, Essigsäure, Butansäure, 1,2-Benzoldicarbonsäure, Ammoniak, Dimethylamin und Diethylamin.

Das erfindungsgemäße Verfahren wird bevorzugt in Anwesenheit eines Lösungsmittels durchgeführt.

Als Lösemittel findet im Allgemeinen das eingesetzte Nucleophil Verwendung, wenn es bei Reaktionsbedingungen als Flüssigkeit vorliegt. Es können jedoch auch andere Lösemittel eingesetzt werden. Die eingesetzten Lösemittel sollten dabei weitgehend inert sein. Bevorzugt wird der Zusatz von Lösemitteln bei Einsatz von Nucleophilen, die unter Reaktionsbedingungen als Feststoffe vorliegen oder bei Produkten, die unter den Reaktionsbedingungen als Feststoffe anfallen würden. Geeignete Lösemittel sind unter anderem aliphatische, cycloaliphatische und aromatische Kohlenwasserstoffe wie zum Beispiel C₃-C₂₀-Alkane, Mischungen niederer Alkane (C₃-C₂₀), Cyclohexan, Cyclooctan, Ethylcyclohexan, Alkene und Polyene, Vinylcyclohexen, 1,3,7-Octatrien, die C₄-Kohlenwasserstoffe aus Crack-C₄-Schnitten, Benzol, Toluol und Xylol; polare Lösemittel wie zum Beispiel tertiäre und sekundäre Alkohole, Amide wie zum Beispiel Acetamid, Dimethylacetamid und Dimethylformamid, Nitrile wie zum Beispiel Acetonitril und Benzonitril, Ketone wie zum Beispiel Aceton, Methylisobutylketon und Diethylketon; Carbonssäureester wie zum Beispiel Essigsäureethylester, Ether wie beispielsweise Dipropylether, Diethylether, Dimethylether, Methyloctylether, 3-Methoxyoctan, Dioxan, Tetrahydrofuran, Anisol, Alkyl- und Arylether von Ethylenglycol, Diethylenglycol und Polyethylenglycol und andere polare Lösemittel wie zum Beispiel Sulfolan, Dimethylsulfoxid, Ethylencarbonat, Propylencarbonat und Wasser. Auch Ionische Flüssigkeiten, beispielsweise Imidazolium oder Pyridiniumsalze, können als Lösemittel eingesetzt werden.

Die Lösemittel kommen allein oder als Mischungen verschiedener Lösemittel bzw. Nucleophile zum Einsatz.

Die Temperatur, bei der die Telomerisationsreaktion ausgeführt wird, liegt zwischen 10 und 180 °C, bevorzugt zwischen 30 und 120 °C, besonders bevorzugt zwischen 40 und 100 °C. Der Reaktionsdruck beträgt 1 bis 300 bar, bevorzugt 1 bis 120 bar, besonders bevorzugt 1 bis 64 bar und ganz besonders bevorzugt 1 bis 20 bar.

Essentiell für das erfindungsgemäße Verfahren ist, dass die Telomerisationsreaktion mit Katalysatoren auf Basis von Metall-Komplexen mit Carbenliganden nach den allgemeinen Formeln (III) bis (VI) durchgeführt wird.

Beispiele für Carbenliganden, die den allgemeinen Formeln (III) bis (VI) entsprechen, und Komplexe, die derartige Liganden enthalten sind zum Teil in der Fachliteratur bereits beschrieben (W. A. Herrmann, C. Köcher, Angew. Chem. 1997, 109, 2257; Angew. Chem. Int. Ed. Engl. 1997, 36, 2162; W.A. Herrmann, T. Weskamp, V.P.W. Böhm, Advances in Organometallic Chemistry, 2001, Vol. 48, 1-69; D. Bourissou, O. Guerret, F. P. Gabbai, G. Bertrand, Chem. Rev. 2000, 100, 39-91).

Für Carbenliganden und Komplexe, die heterocyclische Substituenten tragen, sind allerdings nur wenige Beispiele bekannt (J.C.C. Chen, I.J.B. Lin, Organometallics 2000, 19, 5113).

Das Katalysatormetall der 8. bis 10. Gruppe des Periodensystems kann auf verschiedene Weisen in den Prozess eingebracht werden.
a) Als Metall-Carbenkomplexe
b) In Form von Precursorn, aus denen in situ die Katalysatoren gebildet werden.

### Zu a)

Metall-Carbenkomplexe sind in der Fachliteratur beschrieben (vgl. W. A. Herrmann, C. Köcher, Angew. Chem. 1997, 109, 2257; Angew. Chem. Int. Ed. Engl. 1997, 36, 2162; W.A. Herrmann, T. Weskamp, V.P.W. Böhm, Advances in Organometallic Chemistry, 2001, Vol. 48, 1-69; D. Bourissou, O. Guerret, F. P. Gabbai, G. Bertrand, Chem. Rev. 2000, 100, 39-91; J.C.C. Chen, I.J.B. Lin, Organometallics 2000, 19, 5113) und auf verschiedenen Wegen erhältlich. Beispielsweise können die Komplexe durch Anlagerung von Carbenligand an Metallverbindungen gebildet werden. Dies kann unter Erweiterung der Ligandensphäre erfolgen oder unter Aufbrechen von Brückenstrukturen. Oftmals können aus einfachen Verbindungen der 8. bis 10. Gruppe des Periodensystems, wie Salzen oder Metallkomplexen (Acetate, Acetylacetonate, Carbonyle usw.) durch Umsetzung mit den Carbenliganden Metallverbindungen nach der allgemeinen Formel I erhalten werden. Eine weitere Möglichkeit ist der Austausch von am Zentralmetall koordinierten Liganden durch die Carbenliganden. Dabei werden schwächer koordinierende Liganden (z. B. Solvensmoleküle) durch die Carbenliganden verdrängt.

Im Rahmen dieser Erfindung werden bevorzugt Metall-Carben-Komplexe nach der allgemeinen Formel

[LₐM_{b}X_{c}] [A]ₙ (VII)

eingesetzt,
in der M für Metalle der 8. bis 10. Gruppe des Periodensystems der Elemente steht, X an das Metallatom gebundene ein- oder mehrzähnige geladene oder ungeladene Liganden bedeutet und
A für ein einfach geladenes Anion oder das chemische Äquivalent eines mehrfach geladenen Anions steht, L für einen oder mehrere Liganden der Formeln III bis VI steht, b eine ganze Zahl von 1 bis 3 darstellt, a eine ganze Zahl von 1 bis 4 x b, c = 0 oder eine ganze Zahl von 1 bis 4 x b und n = 0 oder eine ganze Zahl von 1 bis 6 ist.

Die Gruppe A steht bevorzugt für Halogenid-, Sulfat-, Phosphat-, Nitrat-, Pseudohalogenid-, Tetraphenylborat-, Tetrafluoroborat-, Hexafluorophosphat-, und Carboxylat-Ionen, unter den zuletzt genannten bevorzugt das Acetat-Ion, ferner für Metallkomplex-Anionen, beispielsweise Tetrachloropalladat, Tetrachloroaluminat, Tetrachloroferrat(II), Hexafluoroferrat(III), Tetracarbonylcobaltat.

Die ein oder mehrzähnigen Liganden, die in den Komplexen des Fe, Ru, Co, Rh, Ir, Ni, Pd und Pt neben den Carbenliganden enthalten sein können, sind in der allgemeinen Formel (VII) als X widergegeben. X steht für Wasserstoff oder das Wasserstoff-Ion, Halogene oder Halogen-Ionen, Pseudohalogenide, Carboxylat-Ionen, Sulfonat-Ionen, Amidreste, Alkoholatreste, Acetylacetonatrest, Kohlenmonoxid, Alkylreste mit 1 bis 7 Kohlenstoffatomen, Arylreste mit 6 bis 24 Kohlenstoffatomen, Isonitrile, Stickstoffliganden (beispielsweise Stickstoffmonoxid, Nitrile, Amine, Pyridine), Monoolefine, Diolefine, Alkine, Allylgruppen, Cyclopentadienylgruppen, π-Aromaten und Phosphorliganden, die über das Phosphoratom koordinieren. Bei den Phosphorliganden handelt es sich bevorzugt um Verbindungen des dreiwertigen Phosphors, wie Phosphine, Phosphite, Phosphonite, Phosphinite. Sind mehrere Liganden X im Metallkomplex vorhanden, können diese gleich oder verschieden sein.

Tragen die Substituenten der Carbenliganden nach den allgemeinen Formeln (III) bis (VI) funktionelle Gruppen, können diese ebenfalls an das Metallatom koordinieren (chelatisierende Koordination, in der Literatur auch als hemilabile Koordination beschrieben (J.C.C. Chen, I.J.B. Lin, Organometallics 2000, 19, 5113).

### Zu b)

Die Metall-Carbenkomplexe werden in situ aus Vorstufen und Carbenligand bzw. einer Carbenligandenvorstufe gebildet.

Als Vorstufen für die Metallkomplexe der 8. bis 10. Gruppe des Periodensystems kommen beispielsweise Salze oder einfache Komplexverbindungen der Metalle in Frage, beispielsweise Metallhalogenide, Metallacetate, Metallacetylacetonate, Metallcarbonyle.

Zur näheren Erläuterung seien einige konkrete Beispiele für Palladiumverbindunegn angeführt: Palladium(II)acetat, Palladium(II)chlorid, Palladium(II)bromid, Lithiumtetrachloropalladat, Palladium(11)acetylacetonat, Palladium(0)-dibenzylidenaceton-Komplexe, Palladium(II)-propionat, Palladium(II)chloridbisacetonitril, Palladium(II)-bistriphenylphosphandichlorid, Palladium(II)chloridbisbenzonitril, Bis(tri-o-tolylphosphin)palladium(0). Analoge Verbindungen der anderen Metalle der 8. bis 10. Gruppe des Periodensystems können ebenso eingesetzt werden.

Die Carbene nach den allgemeinen Formeln (III) bis (VI) werden in Form freier Carbene oder als Metallkomplexe eingesetzt oder in situ aus Carbenvorstufen erzeugt.

Als Carbenvorstufen eignen sich beispielsweise Salze der Carbene gemäß den allgemeinen Formeln (VIII) bis (XI), 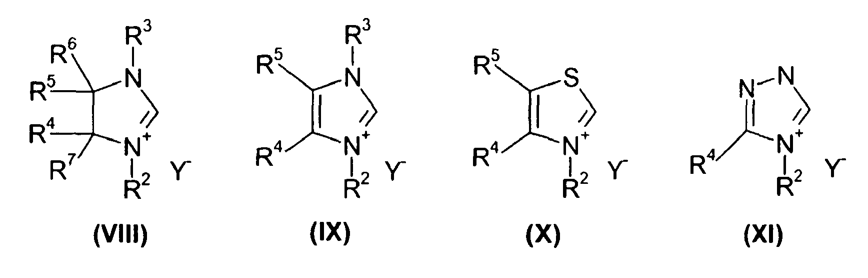 wobei R², R³, R⁴, R⁵, R⁶, R⁷ die bereits genannten Bedeutungen haben und Y für eine einfach geladene anionische Gruppe oder entsprechend der Stöchiometrie anteilig für eine mehrfach geladene anionische Gruppe steht.

Beispiele für Y sind Halogenide, Hydrogensulfat, Sulfat, Phosphat, Alkoholat, Phenolat, Alkylsulfate, Arylsulfate, Borate, Hydrogencarbonat, Carbonat, Alkylcarboxylate, Arylcarboxylate.

Aus den Salzen der Carbene können die entsprechenden Carbene, gegebenenfalls unter Umsetzung mit einer zusätzlichen Base, freigesetzt werden. Als Basen eignen sich beispielsweise Metallhydride, Metallalkoholate, Carbonylmetallate, Metallcarboxylate, Metallamide oder Metallhydroxide.

Die Konzentration des Katalysators, formal angegeben in ppm (Masse) an Katalysatormetall bezogen auf die Gesamtmasse, beträgt 0.01 ppm bis 1000 ppm, bevorzugt 0.5 bis 100 ppm, besonders bevorzugt 1 bis 50 ppm.

Das Verhältnis [Mol/Mol] von Carben zu Metall beträgt 0.01 : bis 250 : 1, bevorzugt 1 : 1 bis 100 : 1, besonders bevorzugt 1 : 1 bis 50 : 1. Neben den Carbenliganden können noch weitere Liganden, beispielsweise Phosphorliganden wie Triphenylphosphin, in der Reaktionsmischung vorliegen.

Aufgrund der Katalysatoraktivitäten und -stabilitäten ist es bei dem erfindungsgemäßen Verfahren möglich, extrem kleine Mengen an Katalysator zu verwenden. Neben einer Verfahrensführung, bei der der Katalysator wiederverwendet wird, wird so auch die Option eröffnet, den Katalysator nicht zu recyceln. Beide Varianten sind in der Patentliteratur bereits beschrieben (WO 90/13531, US 5 254 782, US 4 642 392).

Oftmals ist es vorteilhaft, die Telomerisationsreaktion in Gegenwart von Basen durchzuführen. Bevorzugt werden basische Komponenten mit einem pK_{b}-Wert kleiner 7, insbesondere Verbindungen ausgewählt aus der Gruppe Amine, Alkoholate, Phenolate, Alkalimetallsalze, Erdalkalimetallsalze eingesetzt.

Als basische Komponente sind beispielsweise geeignet Amine wie Trialkylamine, die alicyclisch oder/und offenkettig sein können, Amide, Alkali- oder/und Erdalkalisalze aliphatischer oder/und aromatischer Carbonsäuren, wie Acetate, Propionate, Benzoate bzw. entsprechende Carbonate, Hydrogencarbonate, Alkoholate von Alkali- und/oder Erdalkalielementen, Phosphate, Hydrogenphosphate oder/und Hydroxide bevorzugt von Lithium, Natrium, Kalium, Calcium, Magnesium, Cäsium, Ammonium- und Phosphoniumverbindungen. Bevorzugt sind als Zusatz Hydroxide der Alkali- und Erdalkalielemente und Metallsalze des Nucleophils nach der allgemeinen Formel (II).

Im allgemeinen wird die basische Komponente zwischen 0.01 Mol% und 10 Mol-% (bezogen auf das Olefin), bevorzugt zwischen 0.1 Mol-% und 5 Mol-% und ganz besonders bevorzugt zwischen 0.2 Mol-% und 1 Mol-% eingesetzt.

In dem erfindungsgemäßen Verfahren beträgt das Verhältnis [Mol/Mol] zwischen eingesetztem Dien und Nucleophil 1 : 100 bis 100 : 1, bevorzugt 1 : 50 bis 10 : 1, besonders bevorzugt 1 : 10 bis 2 : 1.

Das erfindungsgemäße Verfahren kann kontinuierlich oder diskontinuierlich betrieben werden und ist nicht auf den Einsatz bestimmter Reaktortypen begrenzt. Beispiele für Reaktoren, in denen die Reaktion durchgeführt werden kann, sind Rührkesselreaktor, Rührkesselkaskade, Strömungsrohr und Schlaufenreaktor. Auch Kombinationen verschiedener Reaktoren sind möglich, beispielsweise ein Rührkesselreaktor mit nachgeschaltetem Strömungsrohr.

Die folgenden Beispiele sollen die Erfindung näher erläutern, ohne den Schutzbereich der Patentanmeldung zu beschränken.

### Beispiele

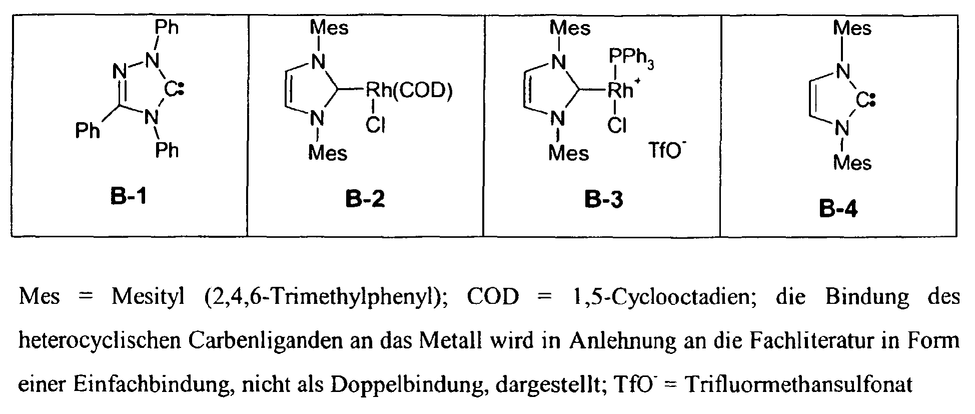

### Beispiel 1 - Telomerisation von 1,3-Butadien mit Methanol

In einem 3-Liter Autoklav (Firma Büchi) wurden 211 g entgastes Methanol, 589 g 1,3-Butadien, 1.20 g Natrimhydroxid, 50 g Cyclooctan (interner GC Standard) und 0.50 g 4-t-Butylcatechol unter Schutzgas vorgelegt und auf 80 °C erwärmt. 0.0494 g Palladiumacetylacetonat und 0.1078 g der Verbindung 5-Methoxy-1,3,4-triphenyl-4,5-dihydro-1H-1,2,4-triazolin (aus der sich unter Abspaltung von Methanol das Carben **B-1** bilden kann) wurden separat unter Schutzgas in 48.4 g entgastem Methanol gelöst. Die Reaktion wurde durch Zugabe dieser Lösung (aus einer Druckbürette) in den Autoklaven gestartet und der Reaktionsverlauf durch gaschromatographische Analyse von regelmäßig entnommenen Proben verfolgt. Nach 180 Minuten waren 18 % des Butadiens umgesetzt, die Selektivität der Reaktion zum 2,7-Octadienyl-1-methylether betrug nach gaschromatographischer Analyse > 96.8 %.

### Beispiel 2

Synthese des Komplexes **B-2:** 60 mg [Rh(COD)Cl]₂ (M = 493.08 g/mol) werden in 2 ml THF (Tetrahydrofuran) gelöst und unter Rühren mit 76 mg des Carbens **B-4** (M = 304.3 g/mol), gelöst in 1 ml THF, bei Raumtemperatur versetzt. Die Lösung wird 3 h gerührt, das THF im Vakuum entfernt, der Niederschlag in CH₂Cl₂ gelöst und filtriert. Das CH₂Cl₂ wird im Vakuum entfernt, der Rückstand mit Pentan gewaschen, abfiltriert und im Vakuum getrocknet. Die Ausbeute beträgt 82 % (110 mg, M = 550.97 g/Mol).

### Beispiel 3

Synthese des Komplexes **B-3:** 113.6 mg **B-2** (0.21 mmol, M = 550.97 g/mol), in 5 ml THF gelöst, werden mit 53 mg AgOTf (0.01 mmol, M = 256.94 g/Mol) und 57 mg PPh₃ (0.21 mmol, M = 262.28 g/ Mol), gelöst in 10 ml THF, bei RT versetzt. Das ausfallende AgCl wird abfiltriert und das THF im Vakuum entfernt. Der Rückstand wird in CH₂Cl₂ aufgenommen, filtriert und das CH₂Cl₂ im Vakuum teilweise entfernt. Der Komplex wird aus wenig CH₂Cl₂ durch Zugabe von Pentan ausgefällt, abfiltriert, mit Pentan gewaschen und im Vakuum getrocknet. Die Ausbeute beträgt 171.8 mg, 90 % ( M = 926.88 g/Mol).

### Beispiele 4 und 5

### Allgemeine Arbeitsvorschrift: zur Telomerisation von Butadien mit Methanol:

In einem 100-ml-Schlenkrohr wird unter Schutzgas eine entsprechende Menge Katalysator in 16.1 g Methanol gelöst. Die Lösung wird mit 1 Mol-% (bezogen auf die eingesetzte Menge an 1,3-Butadien) Natriummethylat (Base) und 5 ml Isooctan (interner GC Standard) versetzt. Anschließend wird die Reaktionslösung in den evakuierten Autoklaven (100 ml Autoklav der Firma Parr) eingesaugt, der Autoklav auf T < -10 °C gekühlt und 13.6 g 1,3-Butadien einkondensiert (Mengenbestimmung durch Massenverlust in der Butadienvorratsflasche). Der Autoklav wird auf Reaktionstemperatur erwärmt und nach 16 Stunden auf Raumtemperatur abgekühlt. Nicht umgesetztes 1,3-Butadien wird in eine mit Trockeneis gekühlte Kühlfalle zurückkondensiert. Der Reaktoraustrag wird gaschromatographisch analysiert.

Die Telomerisation von 1,3-Butadien mit Methanol wurde entsprechend der allgemeinen Arbeitsvorschrift mit den Komplexen B-2 und B-3 durchgeführt. Die Reaktionstemperatur betrug 90 °C.

Als Hauptprodukt der Reaktion wurde 1-Methoxyocta-2,7-dien (n-Produkt) erhalten. Daneben wurde 3-Methoxyocta-1,7-dien (iso-Produkt), 1,3,7-Octatrien (OT), 1,7-Octadien (OD) und Vinylcyclohexen (VCEN) gebildet.

| Bspl. | MeOH : | Kat. | Rh | Base | n+iso | n :iso | OT+OD+VC | TON |
|---|---|---|---|---|---|---|---|---|
| Nr. | Butadien | | [Mol-%] | [Mol-%] | [%] | [%] | H [%] | |
| 4 | 1:2 | B-2 | 0.021 | 1 | 4.6 | 97.7:2.3 | 2.4 | 219 |
| 5 | 1:2 | B-3 | 0.021 | 1 | 1.1 | 95:5 | 2.7 | 52 |

| | | | | | | | | |
|---|---|---|---|---|---|---|---|---|
| n + iso = Ausbeute an n-Produkt und iso-Produkt n : iso = Verhältnis von n-Produkt zu iso-Produkt OT+OD+VCH = Ausbeute an 1,3,7-Octatrien, 1,7-Octadien, Vinylcyclohexen (Summe) TON = Turnover number | | | | | | | | |

## Patentansprüche

1. Verfahren zur katalytischen Telomerisation von 1,3-Butadien mit mindestens einem Nucleophil,
**dadurch gekennzeichnet**,
das Mischungen von 1,3-Butadien mit anderen C₃-, C₄- und/oder C₅-Kohlenwasserstoffen eingesetzt werden, wobei Alkine und gegebenenfalls 1,2 Butadien vor der Telomerisationsrekation entfernt werden, und
dass als Katalysator Komplexe eingesetzt werden, die Metalle der 8. bis 10. Gruppe des Periodensystems der Elemente und mindestens einen Carbenligand nach einer der allgemeinen Formeln 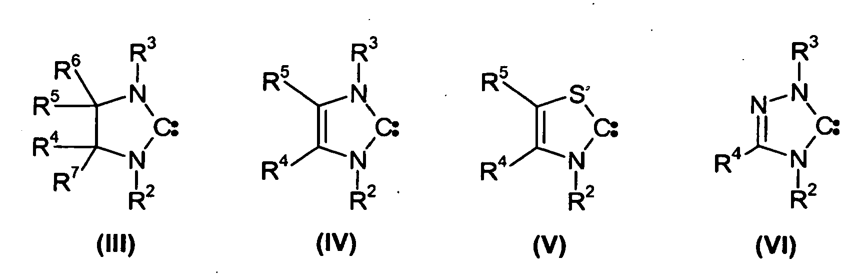 enthalten, mit
R²; R³: gleich oder verschieden a) lineare, verzweigte, substituierte oder unsubstituierte cyclische oder alicyclische Alkylgruppen mit 1 bis 24 Kohlenstoffatomen,
oder b) substituierte oder unsubstituierte, mono- oder polycyclische Arylgruppen mit 6 bis 24 Kohlenstoffatomen
oder c) mono- oder polycyclischer, substituierter oder unsubstituierter Heterocyclus mit 4 bis 24 Kohlenstoffatomen und mindestens einem Heteroatom aus der Gruppe N, O, S
R⁴, R⁵, R⁶, R⁷: gleich oder verschiedenen
Wasserstoff, Alkyl, Heteroaryl, Aryl, -CN, -COOH, -COO-Alkyl-, -COO-Aryl-, -OCO-Alkyl-, -OCO-Aryl-, -OCOO-Alkyl-, -OCOO-Aryl-, -CHO, -CO-Alkyl-, -CO-Aryl-, -O-Alkyl-, -O-Aryl-, -NH₂, -NH(Alkyl)-, -N(Alkyl)₂-, -NH(Aryl)-, -N(Alkyl)₂-, -F, -Cl, -Br, -I, -OH, -CF₃, -NO₂, -Ferrocenyl, -SO₃H, -PO₃H₂, wobei die Alkylgruppen 1 bis 24 und die Arylgruppen 5 bis 24 Kohlenstoffatome beinhalten und die Reste R⁴ und R⁵ auch Teil eines verbrückenden aliphatischen oder aromatischen Ringes sein können,
mit der Maßgabe, das in Kombination mit Pd als Metall der 8. bis 10. Gruppe des Periodensystems R² und/oder R³ die Bedeutung c) hat.

2. Verfahren nach Anspruch 1,
**dadurch gekennzeichnet,**
**dass** R², R³, R⁴, R⁵, R⁶ oder R⁷ jeweils gleich oder verschieden sind und mindestens einen Substituenten aus der Gruppe
-H, -CN, -COOH, -COO-Alkyl, -COO-Aryl, -OCO-Alkyl, -OCO-Aryl, -OCOO-Alkyl, -OCOO-Aryl, -CHO, -CO-Alkyl, -CO-Aryl, -Aryl, -Alkyl, -Alkenyl, -Allyl, -O-Alkyl, -O-Aryl, -NH₂, -NH(Alkyl), -N(Alkyl)₂, -NH(Aryl), -N(Alkyl)₂, -F, -Cl, -Br, -I, -OH, -CF₃, -NO₂, -Ferrocenyl, -SO₃H, -PO₃H₂ aufweisen, wobei die Alkylgruppen 1 bis 24, die Alkenylgruppen 2 bis 24, die Allylgruppen 3 bis 24 und die Arylgruppen 5 bis 24 Kohlenstoffatome beinhalten.

3. Verfahren nach Anspruch 1 oder 2,
**dadurch gekennzeichnet,**
**dass** ein Nukleophil der Formel (II)
R¹-Z-R^{1'} (II)
eingesetzt wird, mit
Z gleich O, N(R^{1"}), S(O₂), Si(R^{1"})(OH), C=O, C(H₂), C(H)(NO₂) oder N(CH₂CH=CH₂) und R¹, R^{1'} oder R^{1"} gleich oder verschieden, H, substituierte oder unsubstituierte, lineare, verzweigte oder cyclische Alkylgruppen, Alkenylgruppen mit 1 bis 22 Kohlenstoffatomen, Carboxylgruppen oder Arylgruppen bedeuten und die Reste R¹, R^{1'} über kovalente Bindungen miteinander verknüpft sein können, wobei R¹ und R^{1"} gleich oder unterschiedlich substituiert sein können,

4. Verfahren nach zumindest einem der Ansprüche 1 bis 3,
**dadurch gekennzeichnet,**
**dass** als Nucleophil Verbindungen der allgemeinen Formel (IIa) oder (IIb)
R¹-O-H (IIa), 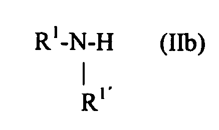
eingesetzt werden,
wobei R¹, R^{1'} jeweils gleich oder verschieden, H, substituierte oder unsubstituierte, lineare, verzweigte oder cyclische Alkylgruppe, eine Alkenylgruppe mit 1 bis 22 Kohlenstoffatomen, einer Carboxylgruppe oder Arylgruppe bedeuten und die Reste R¹, R^{1'} über kovalente Bindungen miteinander verknüpft sein können.

5. Verfahren nach einem der Ansprüche 1 bis 4,
**dadurch gekennzeichnet,**
**dass** als Nucleophil Wasser, Alkohole, Phenole, Polyole, Carbonsäuren, Ammoniak und/oder primäre oder sekundäre Amine eingesetzt werden.

6. Verfahren nach einem der Ansprüche 1 bis 5,
**dadurch gekennzeichnet,**
**dass** das Verfahren in einem Lösemittel durchgeführt wird, wobei das Lösemittel das Nucleophil (II) und/oder inerte organische Lösemittel eingesetzt werden.

7. Verfahren nach einem der Ansprüche 1 bis 6,
**dadurch gekennzeichnet,**
**dass** das Verhältnis von Carbenligand zu Metall [Mol/Mol] 0,01 : 1 bis 250 : 1 beträgt.

## Claims

1. Process for the catalytic telomerization of 1,3-butadiene with at least one nucleophile,
**characterized in that** mixtures of 1,3-butadiene with other C₃-, C₄- and/or C₅-hydrocarbons are used, with alkynes and if appropriate 1,2-butadiene being removed before the telomerization reaction and
**in that** complexes comprising metals of groups 8 to 10 of the Periodic Table of the Elements and at least one carbene ligand having one of the formulae 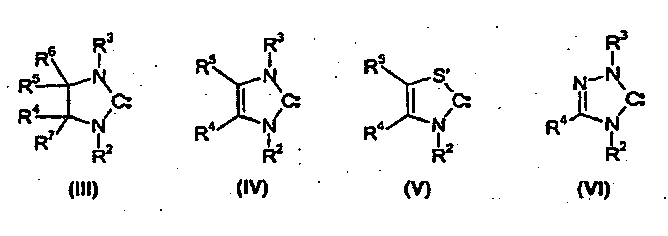 where
R²; R³: are identical or different and are each a) a linear, branched, substituted or unsubstituted cyclic or alicyclic alkyl group having from 1 to 24 carbon atoms, or b) a substituted or unsubstituted, monocyclic or polycyclic aryl group having from 6 to 24 carbon atoms
or c) a monocyclic or polycyclic, substituted or unsubstituted heterocycle having from 4 to 24 carbon atoms and at least one heteroatom from the group consisting of N, O, S,
R⁴, R⁵, R⁶, R⁷: are identical or different and are each hydrogen, alkyl, heteroaryl, aryl, -CN, -COOH, -COO-alkyl, -COO-aryl, -OCO-alkyl, -OCO-aryl, -OCOO-alkyl, -OCOO-aryl, -CHO, -CO-alkyl, -CO-aryl, -O-alkyl, -O-aryl, -NH₂, -NH(alkyl), -N(alkyl)₂, -NH(aryl), -N(alkyl)₂, -F, -Cl, -Br, -I, -OH, -CF₃, -NO₂, -ferrocenyl, -SO₃H, -PO₃H₂, where the alkyl groups have 1-24 carbon atoms and the aryl groups have from 5 to 24 carbon atoms and the radicals R⁴ and R⁵ may also be part of a bridging aliphatic or aromatic ring,
with the proviso that when the metal of groups 8 to 10 of the Periodic Table is Pd, R² and/or R³ have the meaning c), are used as catalyst.

2. Process according to Claim 1, **characterized in that** R², R³, R⁴, R⁵, R⁶ and R⁷ are identical or different and have at least one substituent from the group consisting of -H, -CN, -COOH, -COO-alkyl, -COO-aryl, -OCO-alkyl, -OCO-aryl, -OCOO-alkyl, -OCOO-aryl, -CHO, -CO-alkyl, -CO-aryl, -aryl, -alkyl, -alkenyl, -allyl, -O-alkyl, -O-aryl, -NH₂, -NH(alkyl), -N(alkyl)₂, -NH(aryl), -N(alkyl)₂, -F, -Cl, -Br, -I, -OH, -CF₃, -NO₂, -ferrocenyl, -SO₃H -PO₃H₂, where the alkyl groups have from 1 to 24 carbon atoms, the alkenyl groups have from 2 to 24 carbon atoms, the allyl groups have from 3 to 24 carbon atoms and the aryl groups have from 5 to 24 carbon atoms.

3. Process according to Claim 1 or 2, **characterized in that** a nucleophile of the formula (II)
R¹-Z-R^{1'} (II),
where
Z is O, N(R^{1"}) S(O₂), Si(R^{1"}) (OH) , C=O, C(H₂), C(H)(NO₂) or N(CH₂CH=CH₂) and R¹, R^{1'} or R^{1"} are identical or different and are each H, a substituted or unsubstituted, linear, branch or cyclic alkyl group, alkenyl group having from 1 to 22 carbon atoms, carboxyl group or aryl group and the radicals R¹, R^{1'} may be joined to one another via covalent bonds, where R¹ and R^{1'} may be identical or different, is used.

4. Process according to at least one of Claims 1 to 3, **characterized in that** compounds of the formula (IIa) or (IIb)
R¹- O-H (IIa), 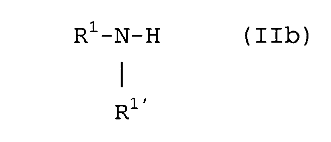
where R¹, R^{1'} are identical or different and are each H, a substituted or unsubstituted, linear, branched or cyclic alkyl or alkenyl group having from 1 to 22 carbon atoms, a carboxyl group or an aryl group and the radicals R¹, R^{1'} may be joined to one another via covalent bonds, are used as nucleophile.

5. Process according to any of Claims 1 to 4, **characterized in that** water, alcohols, phenols, polyols, carboxylic acids, ammonia and/or primary or secondary amines are used as nucleophile.

6. Process according to any of Claims 1 to 5, **characterized in that** the process is carried out in a solvent, where the nucleophile (II) and/or inert organic solvents is/are used as solvent.

7. Process according to any of Claims 1 to 6, **characterized in that** the ratio of carbene ligand to metal [mol/mol] is from 0.01:1 to 250:1.

## Revendications

1. Procédé de télomérisation catalytique de buta-1,3-diène avec au moins un nucléophile,
**caractérisé en ce que**
l'on met en oeuvre des mélanges de buta-1,3-diène avec d'autres hydrocarbures C₃, C₄ et/ou C₅, les alcynes et le cas échéant le buta-1,2-diène étant éliminés en amont de la réaction de télomérisation, et
l'on met en oeuvre, en tant que catalyseur, des complexes contenant des métaux des groupes 8 à 10 du tableau périodique des éléments et au moins un ligand carbénique selon une des formules générales suivantes 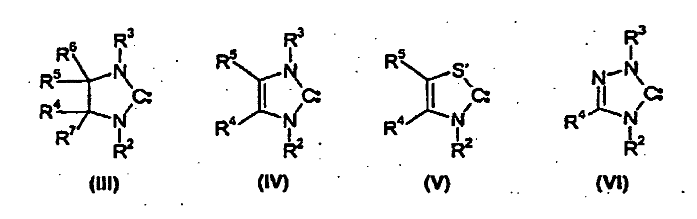 dans lesquelles
R² ; R³ : identiques ou différents, sont a) des groupes alkyle cycliques ou alicycliques, linéaires, ramifiés, substitués ou non-substitués renfermant 1 à 24 atomes de carbone,
ou b) des groupes aryle mono- ou polycycliques, substitués ou non-substitués, renfermant 6 à 24 atomes de carbone
ou c) un hétérocycle mono- ou polycyclique, substitué ou non-substitué, renfermant 4 à 24 atomes de carbone et au moins un hétéroatome choisi parmi le groupe N, O, S
R⁴, R⁵, R⁶, R⁷ : identiques ou différents, sont de l'hydrogène, alkyle, hétéroaryle, aryle, -CN, -COOH, -COO-alkyl-, -COO-aryl-, -OCO-alkyl-, -OCO-aryl-, -OCOO-alkyl-, -OCOO-aryl-, -CHO, -CO-alkyl-, -CO-aryl-, -O-alkyl-, -O-aryl-, -NH₂, -NH(alkyl)-, -N(alkyl)₂-, -NH(aryl)-, -N(alkyl)₂-, -F, -Cl, -Br, -I, -OH, -CF₃, -NO₂, -ferrocényle, -SO₃H, -PO₃H₂, les groupes alkyle renfermant 1 à 24 et les groupes aryle renfermant 5 à 24 atomes de carbone et les radicaux R⁴ et R⁵ pouvant également faire partie d'un cycle aliphatique ou aromatique de pontage,
à condition que, lorsque ledit métal des groupes 8 ou 10 du tableau périodique est du Pd, R² et/ou R³ aient la signification c).

2. Procédé selon la revendication 1,
**caractérisé en ce que**
R², R³, R⁴, R⁵, R⁶ ou R⁷ sont identiques ou différents l'un de l'autre et comportent au moins un substituant issu du groupe -H, -CN, -COOH, -COO-alkyle, -COO-aryle, -OCO-alkyle, -OCO-aryle, -OCOO-alkyle, -OCOO-aryle, -CHO, -CO-alkyle, -CO-aryle, -aryle, -alkyle, -alcényle, -Allyle, -'-alkyle, -'-aryle, -NH₂, -NH(alkyle), -N(alkyle)₂, -NH(aryle), -N(alkyle)₂, -F, -Cl, -Br, -I, -OH, -CF₃, -NO₂, -ferrocényle, -SO₃H -PO₃H₂ aufweisen, les groupes alkyle renfermant 1 à 24, les groupes alcényle 2 à 24, les groupes allyle 3 à 24 et les groupes aryle 5 à 24 atomes de carbone.

3. Procédé selon les revendications 1 ou 2,
**caractérisé en ce que**
l'on met en oeuvre un nucléophile de formule (II)
R¹-Z-R^{1'} (II)
dans laquelle
Z égale O, N(R^{1"}), S(O₂), Si(R^{1"})(OH) , C=O, C(H₂), C(H)(NO₂) ou N(CH₂CH=CH₂) et R¹, R^{1'} ou R^{1"}, identiques ou différents, représentent H, des groupes alkyle ou des groupes alcényle linéaires, ramifiés ou cycliques, substitués ou non-substitués, renfermant 1 à 22 atomes de carbone, des groupes carboxyle ou des groupes aryle, les radicaux R¹, R^{1'} pouvant être liés l'un à l'autre par des liaisons covalentes, R¹ et R^{1'} pouvant être substitués de façon identique ou différente.

4. Procédé selon au moins une des revendications 1 à 3,
**caractérisé en ce que**
l'on met en oeuvre, en tant que nucléophile, des composés répondant aux formules générales (IIa) ou (IIb)
R¹-O-H (IIa), 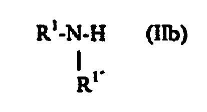
R¹, R^{1'}, identiques ou différents l'un de l'autre, représentant H, un groupe alkyle linéaire, ramifié
ou cyclique substitué ou non-substitué, un groupe alcényle avec 1 à 22 atomes de carbone, un groupe carboxyle ou un groupe aryle, les radicaux R¹, R^{1'} pouvant être liés l'un à l'autre par des liaisons covalentes.

5. Procédé selon l'une des revendications 1 à 4,
**caractérisé en ce que**
l'on met en oeuvre, en tant que nucléophile, de l'eau, des alcools, des phénols, des polyols, des acides carboxyliques, de l'ammoniaque et/ou des amines primaires ou secondaires.

6. Procédé selon l'une des revendications 1 à 5,
**caractérisé en ce qu'**
il est réalisé dans un solvant, le solvant mis en oeuvre correspondant au nucléophile (II) et/ou à des solvants organiques inertes.

7. Procédé selon l'une des revendications 1 à 6,
**caractérisé en ce que**
le rapport entre le ligand carbénique et le métal [mole/mole] est compris entre 0,01 : 1 et 250 : 1.
